# EUROPEAN PATENT SPECIFICATION

(11) **EP 0 756 484 B1**
(45) Date of publication and mention of the grant of the patent: **09.10.2002**
(21) Application number: 95917120.8
(22) Date of filing: 24.04.1995
(51) Int. Cl.: A61K 31/66, A61K 9/20

(54) **WET GRANULATION FORMULATION FOR BISPHOSPHONIC ACIDS**
FEUCHTGRANULIERUNG FORMULIERUNG FÜR BISPHOSPHONSÄUREN
COMPOSITION POUR ACIDES BISPHOSPHONIQUES A GRANULATION PAR VOIE HUMIDE

(30) Priority: 29.04.1994 US 236904
(43) Date of publication of application: 05.02.1997
(73) Proprietor: Merck & Co., Inc., Rahway New Jersey 07065-0900 (US)
(72) Inventor: KATDARE, Ashok, V., Rahway, NJ 07065 (US); KRAMER, Kenneth, A., Rahway, NJ 07065 (US)
(74) Representative: Hiscock, Ian James
(86) International application number: US9504965
(87) International publication number: WO95029679

(56) References cited:
- EP-A- 0 177 443
- EP-A- 0 282 320
- EP-A- 0 336 851
- EP-A- 0 421 921
- EP-A- 0 566 535
- EP-A- 0 623 347
- WO-A-92/11269
- WO-A-93/21907
- WO-A-94/12200
- FR-A- 2 259 615

## Description

### BACKGROUND OF THE INVENTION

The pharmaceutical industry employs various methods for compounding pharmaceutical agents in tablet formulations. In particular, wet granulation is one of the most prevalent methods. Tablets prepared by wet granulation generally require the addition of a binding agent to keep the tablet together.

A variety of bisphosphonic acids have been disclosed as being useful in the treatment and prevention of diseases involving bone resorption. Representative examples may be found in the following:
U.S. Patent No. 3,962,432; U.S. Patent No. 4,054,598;
U.S. Patent No. 4,267,108; U.S. Patent No. 4,327,039;
U.S. Patent No. 4,621,077; U.S. Patent No. 4,624,947;
U.S. Patent No. 4,746,654; U.S. Patent No. 4,922,007; and EPO Patent Pub. No. 0,252,504. Standard methods for tablet formulation of bisphosphonic acids, however, suffer difficulties.

US-A-5344825 (Ciba-Geigy Corp.) discloses a sustained release dosage form comprising a methanediphosphonic acid derivative and a cationic macroporous ion exchange resin that, together with customary excipients including a binder, may be directly compressed into a tablet. Alternatively granules comprising the active ingredient and resin together with customary excipients including a binder may be compressed into a tablet.

US-A-5096717 (Ciba-Geigy Corporation) discloses double-coated granules comprising disodium pamidronate and adjuncts which are customary in pharmaceutical technology for tableting methods, including a binder. The granules can be compressed to tablets.

US-A-4711880 (Ciba-Geigy Corporation and Henkel Kommanditgesellschaft auf Akten) discloses granules comprising disodium pamidronate and a binder, such as wheat starch, which are compressed to form tablets.

WO-A-9412200 (Merck & Co., Inc.) which is prior art for novelty purposes only discloses a wet granulation process for making a tablet wherein the active ingredient is 5mg equivalent of 4-amino-1-hydroxybutylidene-1,1-bisphosphonic acid, the diluents are anhydrous lactose and microcrystalline cellulose, and drying is carried out in a forced air dryer. The resulting granules are mixed with croscarmellose sodium and magnesium stearate.

Wet granulated formulations need to have an agent called a "binder," which, in contact with water, swells or starts dissolving, forming a gel-like consistency. Traditionally, starch, starch paste, gelatin, and cellulosics such as hydroxypropylmethyl cellulose, hydroxyethyl cellulose, hydroxypropyl cellulose, polyvinyl pyrrolidone are used as binding agents in wet granulation formulations. (See, Remington's Pharmaceutical Sciences, 18th ed, (Mack Publishing Company: Easton, PA, 1990), pp.1635-36). Microcrystalline cellulose, such as Avicel PH101, may be employed as a binder or compression aid in compositions prepared by dry granulation formulation, but microcrystalline cellulose functions primarily as a bulking agent in wet granulation formulations because the microcrystalline cellulose loses much of its binding properties upon wetting.

The wet granulation process helps to form agglomerates of powders. These agglomerates are called "granules." The present invention provides for a wet granulated formulation of bisphosphonic acids and process therefor wherein the tablet formulation does not contain any binder. Instead, the drug itself acts as a binder. The absence of a separate binder keeps the formulation simpler, and minimizes adverse effects that binding agents can have on dissolution. Elimination of binder also simplifies the optimization and characterization of the formulation.

### DESCRIPTION OF THE INVENTION

The present invention is directed in a first embodiment to a process for the preparation of pharmaceutical compositions of bisphosphonic acids by wet granulation formulation. This process employs a blend of a bisphosphonic acid and minimal amounts of other processing aids with no binder added. This tablet formulation in which the active ingredient is the binder is prepared by:
(1) forming a powder blend of the active ingredient with diluents,
(2) wet granulating the powder blend with water to form granules,
(3) drying the granules to remove water, and
(4) compressing the lubricated granule mixture into a desired tablet form; with the proviso that when the active ingredient is 5mg equivalent of 4-amino-1-hydroxy butylidene-1,1-bisphosphonic acid, the diluents are anhydrous lactose and microcrystalline cellulose and the drying step is carried out in a forced air dryer then the resulting granules are not mixed with croscarmellose sodium and magnesium stearate.
The shape of the tablet is not critical.

More specifically, this embodiment of the present invention concerns a process for the preparation of a tablet containing a bisphosphonic acid as an active ingredient which process comprises:
(1) forming a powder blend of the active ingredient with diluents from 3 to 25 minutes using a mixer such as a planetary or high shear granulator,
(2) wet granulating the powder blend by the addition of water while mixing over a 2 to 30 minute period to form granules,
(3) drying the granules to remove water by the use of heated air for 10 minutes to 24 hours in a dryer (fluid bed or tray type),
(4) milling the dried granules to a uniform size,
(5) adding and blending a disintegrant with the dried milled particles for 2 to 30 minutes,
(6) adding and blending a lubricant to the mixture containing the disintegrant for 30 seconds to 20 minutes, and
(7) compressing the lubricated granule mixture into a desired tablet form.

One particularly preferred process employs a high shear granulator as a mixer and comprises the steps of:
(1) forming a powder blend of 4-amino-1-hydroxy-butylidene-1,1-bisphosphonic acid, microcrystalline cellulose, such as Avicel PH101, and lactose with a high shear granulator for 3 to 5 minutes ,
(2) wet granulating the powder blend by the addition of water while mixing over a 3 to 5 minute period to form granules with the high shear granulator,
(3) drying the granules to remove water by the use of heated air by drying 10 minutes to 1 hour with a fluid bed, or 12-24 hours in a tray dryer, preferably with a fluid bed,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender or a planetary mixer for 3 to 8 minutes, and
(7) compressing the lubricated granule mixture into a desired tablet form, and
(8) dedusting and storing the tablets.

Another particularly preferred process employs a planetary granulator as a mixer and comprises the steps of:
(1) forming a powder blend of 4-amino-1-hydroxy-butylidene-1,1-bisphosphonic acid, microcrystalline cellulose such as Avicel PH101, and lactose with a planetary granulator for 10 to 25 minutes ,
(2) wet granulating the powder blend by the addition of water while mixing over a 3 to 10 minute period to form granules with the planetary granulator,
(3) drying the granules to remove water by the use of heated air by drying 10 minutes to 1 hour with a fluid bed, or 12-24 hours in a tray dryer, preferably with a fluid bed,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender or a planetary granulator for 3 to 8 minutes, and
(7) compressing the lubricated granule mixture into a desired tablet form, and
(8) dedusting and storing the tablets.

Still another particularly preferred process employs a high shear granulator as mixer, and comprises the steps of:
(1) forming a powder blend of 4-amino-1-hydroxy-butylidene-1,1-bisphosphonic acid, microcrystalline cellulose, such as Avicel PH101, and lactose with a high shear granulator for 3 to 5 minutes,
(2) wet granulating the powder blend by the addition of water while mixing over a 3 to 5 minute period to form granules with a high shear granulator,
(3) drying the granules to remove water by the use of heated air for 10 minutes to one hour using a fluid bed dryer,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender for 3 to 8 minutes,
(7) compressing the lubricated granule mixture into a desired tablet form, and
(8) dedusting and storing the tablets.

Granulation is the process of adding water to a powder mixture with mixing until granules are formed. The granulation step may be varied from 2 to 30 minutes, preferably 2 to 5 minutes. The lubrication step is the process of adding lubricant to the mixture; the lubrication step may be varied from 30 seconds to 20 minutes, preferably 3 to 8 minutes.

The disclosed process is used to prepare solid dosage forms, namely tablets, for medicinal administration.

Preferred diluents include: lactose, microcrystalline cellulose, calcium phosphate(s), mannitol, powdered cellulose, pregelatinized starch, and other suitable diluents. Especially preferred are lactose and microcrystalline cellulose. In particular, microcrystalline cellulose NF, especially Avicel PH101, the trademarked name for microcrystalline cellulose NF manufactured by FMC Corp. is preferred.

The disintegrant may be one of several modified starches or modified cellulose polymers, in particular, croscarmellose sodium is preferred. Croscarmellose sodium NF Type A is commercially available under the trade name "Ac-di-sol".

Preferred lubricants include magnesium stearate, calcium stearate, stearic acid, surface active agents such as sodium lauryl sulfate, propylene glycol, sodium dodecane sulfonate, sodium oleate sulfonate, and sodium laurate mixed with stearates and talc, sodium stearyl fumarate, and other known lubricants. Especially preferred is magnesium stearate.

The bisphosphonic acids which may be employed as active ingredients in the instant invention are:
(a) 4-amino-1-hydroxybutylidene-1,1-bisphosphonic acid;
(b) N-methyl-4-amino-1-hydroxybutylidene-1,1-bisphosphonic acid;
(c) 4-(N,N-dimethylamino)-1-hydroxybutylidene-1,1-bisphosphonic acid;
(d) 3-amino-1-hydroxypropylidene-1,1-bisphosphonic acid;
(e) 3-(N,N-dimethylamino)-1-hydroxypropylidene-1,1-bisphosphonic acid;
(f) 1-hydroxy-3-(N-methyl-N-pentylamino)propylidene-1,1-bisphosphonic acid;
(g) 1-hydroxy-2-[3-pyridyl]ethylidene-1,1-bisphosphonic acid; and
(h) 4-(hydroxymethylene-1,1-bisphosphonic acid)-piperidine;
or pharmaceutically acceptable salts thereof.

Methods for the preparation of bisphosphonic acids may be found in, e.g., U.S. Patent No. 3,962,432; U.S. Patent No. 4,054,598; U.S. Patent No. 4,267,108; U.S. Patent No. 4,327,039; U.S. Patent No. 4,407,761; U.S. Patent No. 4,621,077; U.S. Patent No. 4,624,947; U.S. Patent No. 4,746,654; U.S. Patent No. 4,922,007; and EPO Patent Pub. No. 0,252,504. In particular, methods for the preparation of 4-amino-1-hydroxy-butylidene-1,1-bisphosphonic acid and 4-amino-1-hydroxy-butylidene-1,1-bisphosphonic acid monosodium salt trihydrate may be found in U.S. Patent No. 4,407,761 and U.S. Patent No. 4,922,007, respectively.

The pharmaceutically acceptable salts of bisphosphonic acids may also be employed in the instant invention. Examples of base salts of bisphosphonic acids include ammonium salts, alkali metal salts such as potassium and sodium (including mono-, di- and tri-sodium) salts (which are preferred), alkaline earth metal salts such as calcium and magnesium salts, salts with organic bases such as dicyclohexylamine salts, N-methyl-D-glucamine, and salts with amino acids such as arginine, lysine, and so forth. The non-toxic, physiologically acceptable salts are preferred. The salts may be prepared by methods known in the art, such as in U.S. Patent No. 4,922,007.

In the present invention it is preferred that the bisphosphonic acid is 4-amino-1-hydroxybutylidene-1,1-bisphosphonic acid. It is even more preferred that the bisphosphonic acid is a sodium salt of 4-amino-1-hydroxybutylidene-1,1-bisphosphonic acid, in particular, 4-amino-1-hydroxybutylidene-1,1-bisphosphonic acid monosodium salt trihydrate.

Preferred pharmaceutical compositions comprise 0.5 to 25% by weight of a bisphosphonic acid as an active ingredient; 30 to 70% by weight of anhydrous lactose or hydrous fast flow lactose; 30 to 50% by weight of microcrystalline cellulose; and 0.1 to 2% by weight of magnesium stearate.

The pharmaceutical compositions are in the form of tablets. The tablets may be, for example, from 50 mg to 1.0 g in net weight, more preferably 100 to 500 mg net weight, and even more preferably 150 to 300 mg net weight.

More preferred pharmaceutical compositions in accordance with the present invention comprise: 0.5 to 25% by weight of a bisphosphonic acid selected from 4-amino-1-hydroxybutylidene-1,1-bisphosphonic acid and 4-amino-1-hydroxybutylidene-1,1-bisphosphonic acid monosodium salt trihydrate; 30 to 70% by weight of anhydrous lactose or hydrous fast flow lactose; 30 to 50% by weight of microcrystalline cellulose; and 0.1 to 2% by weight of magnesium stearate do not further comprise croscarmellose sodium.

Preferred pharmaceutical compositions as envisioned for commercial development are as follows.

Tablets of 2.5 mg potency free acid:
about 1.63% by weight of 4-amino-1-hydroxy- butylidene-1,1-bisphosphonic acid monosodium salt trihydrate; about 56.87% by weight of anhydrous lactose; about 40% by weight of microcrystalline cellulose; about 1 % by weight of croscarmellose sodium; and about 0.5% by weight of magnesium stearate.

Tablets of 5 mg potency free acid:
about 3.25% by weight of 4-amino-1-hydroxy- butylidene-1,1-bisphosphonic acid monosodium salt trihydrate; about 55.25% by weight of anhydrous lactose; about 40% by weight of microcrystalline cellulose; about 1 % by weight of croscarmellose sodium; and about 0.5% by weight of magnesium stearate.

Tablets of 10 mg potency free acid:
about 6.5% by weight of 4-amino-1-hydroxy- butylidene-1,1-bisphosphonic acid monosodium salt trihydrate; about 52.0% by weight of anhydrous lactose; about 40% by weight of microcrystalline cellulose; about 1 % by weight of croscarmellose sodium; and about 0.5% by weight of magnesium stearate.

Tablets of 20 mg potency free acid:
about 13.0% by weight of 4-amino-1-hydroxy- butylidene-1.1-bisphosphonic acid monosodium salt trihydrate; about 45.5% by weight of anhydrous lactose; about 40% by weight of microcrystalline cellulose; about 1 % by weight of croscarmellose sodium; and about 0.5% by weight of magnesium stearate.

Tablets of 40 mg potency free acid:
about 26.0% by weight of 4-amino-1-hydroxy-butylidene-1,1-bisphosphonic acid monosodium salt trihydrate; about 32.5% by weight of anhydrous lactose; about 40% by weight of microcrystalline cellulose; about 1 % by weight of croscarmellose sodium; and about 0.5% by weight of magnesium stearate.

Each of the tablets of the potencies above is preferably formulated in a 200 mg tablet containing 0.05 mL purified water USP per tablet.

The pharmaceutical tablet compositions of the present invention may also contain one or more additional formulation ingredients may be selected from a wide variety of excipients known in the pharmaceutical formulation art. According to the desired properties of the tablet, any number of ingredients may be selected, alone or in combination, based upon their known uses in preparing tablet compositions. Such ingredients include, but are not limited to, diluents, compression aids, disintegrants, lubricants, flavors, flavor enhancers, sweetener and preservatives. The pharmaceutical tablet compositions of the present invention do not, however, require the addition of a separate binding excipient because in wet granulation the active ingredient itself acts as a binding agent.

The term "tablet" as used herein is intended to encompass compressed pharmaceutical dosage formulations of all shapes and sizes, whether coated or uncoated. Substances which may be used for coating include hydroxypropylmethylcellulose, hydroxypropylcellulose, titanium dioxide, talc, sweeteners, and colorants.

The pharmaceutical compositions of the present invention are useful in the therapeutic or prophylactic treatment of disorders in calcium or phosphate metabolism and associated diseases. These diseases can be divided into two categories:
1. Abnormal (ectopic) depositions of calcium salts, mostly calcium phosphate, pathological hardening of tissues and bone malformations.
2. Conditions which can benefit from a reduction in bone resorption. A reduction in bone resorption should improve the balance between resorption and formation, reduce bone loss or result in bone augmentation. A reduction in bone resorption can alleviate the pain associated with osteolytic lesions and reduce the incidence and/or growth of those lesions.

These diseases include: osteoporosis (including estrogen deficiency, immobilization, glucocorticoid induced and senile), osteodystrophy, Paget's disease, myositis ossificans, Bechterew's disease, malignant hypercalcaemia, metastatic bone disease, peridontal disease, cholelithiasis, nephrolithiasis, urolithiasis, urinary calculus, hardening of the arteries (sclerosis), arthritis, bursitis, neuritis and tetany.

Increased bone resorption can be accompanied by pathologically high calcium and phosphate concentrations in the plasma, which would be alleviated by use of the instant pharmaceutical compositions.

The following examples are given for the purpose of illustrating the present invention.

### EXAMPLE 1

### Procedure for Manufacturing 2.5 mg Potency Tablets of 4-Amino-1-hydroxybutylidene-1,1-bisphosphonic acid

| Ingredients | Per Tablet | Per 10,000 Tablets |
|---|---|---|
| Active ingredient (monosodium salt trihydrate) | 3.26 mg | 32.6 g |
| | | |
| Anhydrous Lactose, NF | 113.74 mg | 1137.4 g |
| | | |
| Microcrystalline Cellulose NF | 80.0 mg | 800.0 g |
| | | |
| Magnesium Stearate Impalpable Powder NF | 1.00 mg | 10.0 g |
| | | |
| Croscarmellose Sodium NF Type A | 2.00 mg | 20.0 g |

The active ingredient (equivalent to 2.5 mg anhydrous free acid per tablet) was mixed with the microcrystalline cellulose NF and the anhydrous lactose NF in a high shear mixer for 3 minutes. Granulating solvent (550 mL water) was added to this blend with the mixer running over a two minute period. The wetted mass was dried in a fluid bed dryer at an inlet temperature of 50°C. The dried material was then milled using a FITZPATRICK J mill (hammer-type mill) to achieve fine granules. After milling, Croscarmellose Sodium NF type A (disintegrant) was added to the blend and mixed in a ribbon blender for 5 minutes. Magnesium Stearate Impalpable Powder NF (lubricant) was added to this blend through a #60 mesh screen and blended for an additional 4 minutes. The lubricated mixture was compressed to provide tablets of 2.5 mg active ingredient.

### EXAMPLE 2

### Procedure for Manufacturing 10 mg Potency Tablets of 4-Amino-1-hydroxybutylidene-1,1-bisphosphonic acid

| Ingredients | Per Tablet | Per 10,000 Tablets |
|---|---|---|
| Active ingredient (monosodium salt trihydrate) | 13.05 mg | 130.5 g |
| Anhydrous Lactose, NF | 103.95 mg | 1039.5 g |
| Microcrystalline Cellulose NF | 80.0 mg | 800.0 g |
| Magnesium Stearate Impalpable Powder NF | 1.00 mg | 10.0 g |
| Croscarmellose Sodium NF Type A | 2.00 mg | 20.0 g |

Tablets were prepared using essentially the procedure of Example 1.

### EXAMPLE 3

### Procedure for Manufacturing 20 mg Potency Tablets of 4-Amino-1-hydroxybutylidene-1,1-bisphosphonic acid

| Ingredients | Per Tablet | Per 10,000 Tablets |
|---|---|---|
| Active ingredient (monosodium salt trihydrate) | 26.11 mg | 261.1 g |
| Anhydrous Lactose, NF | 90.89 mg | 908.9 g |
| Microcrystalline Cellulose NF | 80.0 mg | 800.0 g |
| Magnesium Stearate Impalpable Powder NF | 1.0 mg | 10.0 g |
| Croscarmellose Sodium NF Type A | 2.0 mg | 20.0 g |

Tablets were prepared using essentially the procedure of Example 1.

## Claims

1. A process for the preparation of a tablet containing an active ingredient which is the binder selected from the group consisting of:
4-amino-1-hydroxybutylidene-1,1-bisphosphonic acid;
N-methyl-4-amino-1-hydroxybutylidene-1,1-bisphosphonic acid;
4-(N,N-dimethylamino)-1-hydroxybutylidene-1,1-bisphosphonic acid;
3-amino-1-hydroxypropylidene-1,1-bisphosphonic acid;
3-(N,N-dimethylamino)-1-hydroxypropylidene-1,1-bisphosphonic acid;
1-hydroxy-3-(N-methyl-N-pentylamino)propylidene-1,1-bisphosphonic acid;
1-hydroxy-2-[3-pyridyl]ethylidene-1,1-bisphosphonic acid; and
4-(hydroxymethylene-1,1-bisphosphonic acid)piperidine;
or a pharmaceutically acceptable salt thereof;
which process comprises:
(1) forming a powder blend of the active ingredient with diluents,
(2) wet granulating the powder blend with water to form granules,
(3) drying the granules to remove water, and
(4) compressing the dried granules mixture into a desired tablet form;
with the proviso that when the active ingredient is 5 mg equivalent of 4-amino-1-hydroxybutylidene-1,1-bisphosphonic acid, the diluents are anhydrous lactose and microcrystalline cellulose and the drying step is carried out in a forced air dryer then the resulting granules are not mixed with croscarmellose sodium and magnesium stearate.

2. The process of Claim 1 wherein the active ingredient is 4-amino-1-hydroxybutylidene-1,1-bisphosphonic acid.

3. The process of Claim 1 wherein the active ingredient is 4-amino-1-hydroxybutylidene-1,1-bisphosphonic acid monosodium salt.

4. The process of Claim 1 wherein the active ingredient is 4-amino-1-hydroxybutylidene-1,1-bisphosphonic acid monosodium salt trihydrate.

5. A process according to Claim 1, 2 or, 3 or 4
which process comprises:
(1) forming a powder blend of the active ingredient with diluents from 3 to 25 minutes using a mixer such as a planetary or high shear granulator,
(2) wet granulating the powder blend by the addition of water while mixing over a 2 to 30 minute period to form granules,
(3) drying the granules to remove water by the use of heated air for 10 minutes to 24 hours,
(4) milling the dried granules to a uniform size,
(5) adding and blending a disintegrant with the dried milled particles for 2 to 30 minutes,
(6) adding and blending a lubricant to the mixture containing the disintegrant for 30 seconds to 20 minutes, and
(7) compressing the dried granules mixture into a desired tablet form.

6. The process of Claim 5 wherein the diluents are selected from: lactose, microcrystalline cellulose, calcium phosphate, mannitol, powdered cellulose, and pregelatinized starch.

7. The process of Claim 6 wherein the diluents are lactose and microcrystalline cellulose.

8. The process of Claim 7 wherein the lactose is lactose NF anhydrous and the microcrystalline cellulose is Avicel PH101.

9. The process of Claim 5 wherein the disintegrant is selected from the group consisting of modified starch, modified cellulose polymer, and croscarmellose sodium, or a combination thereof.

10. The process of Claim 9 wherein the disintegrant is croscarmellose sodium.

11. The process of Claim 10 wherein the disintegrant is croscarmellose sodium NF type A.

12. The process of Claim 5 wherein the lubricant is selected from the group consisting of magnesium stearate, calcium stearate, stearic acid, sodium lauryl sulfate, propylene glycol, sodium dodecane sulfonate, sodium oleate sulfonate, sodium laurate mixed with stearates and talc, and sodium stearyl fumarate.

13. The process of Claim 12 wherein the lubricant is magnesium stearate.

14. The process of Claim 5 which comprises the steps:
(1) forming a powder blend of 4-amino-1-hydroxybutylidene-1,1-bisphosphonic acid, microcrystalline cellulose, and lactose with a high shear granulator for 3 to 5 minutes,
(2) wet granulating the powder blend by the addition of water while mixing over a 3 to 5 minute period to form granules with the high shear granulator,
(3) drying the granules to remove water by the use of heated air by drying 10 minutes to 1 hour with a Fluid bed, or 12 to 24 hours in a tray dryer,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender or a planetary mixer for 3 to 8 minutes,
(7) compressing the lubricated granule mixture into a desired tablet form, and
(8) dedusting and storing the tablets.

15. The process of Claim 5 which comprises the steps:
(1) forming a powder blend of 4-amino-1-hydroxybutylidene-1,1-bisphosphonic acid, microcrystalline cellulose, and lactose with a planetary granulator for 10 to 25 minutes
(2) wet granulating the powder blend by the addition of water while mixing over a 3 to 10 minute period to form granules with the planetary granulator,
(3) drying the granules to remove water by the use of heated air by drying 10 minutes to 1 hour with a fluid bed, or 12-24 hours in a tray dryer,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender or a planetary granulator for 3 to 8 minutes, and
(7) compressing the lubricated granule mixture into a desired tablet form, and
(8) dedusting and storing the tablets.

16. The process of Claim 5 which comprises the steps:
(1) forming a powder blend of 4-amino-1-hydroxybutylidene-1,1-bisphosphonic acid, Avicel PH101 microcrystalline cellulose, and lactose with a high shear granulator for 3 to 5 minutes,
(2) wet granulating the powder blend by the addition of water while mixing over a 3 to 5 minute period to form granules with a high shear granulator,
(3) drying the granules to remove water by the use of heated air for 10 minutes to one hour using a fluid bed dryer,
(4) milling the dried granules to a uniform size using a hammer type mill,
(5) adding and blending the disintegrant croscarmellose sodium NF type A with the dried milled particles for 3 to 8 minutes,
(6) adding and blending magnesium stearate lubricant to the mixture containing the croscarmellose sodium NF type A disintegrant with a ribbon blender for 3 to 8 minutes,
(7) compressing the lubricated granule mixture into a desired tablet form, and
(8) dedusting and storing the tablets.

17. A solid dosage form containing an active ingredient elected from the group consisting of:
4-amino-1-hydroxybutylidene-1,1-bisphosphonic acid;
N-methyl-4-amino-1-hydroxybutylidene-1,1-bisphosphonic acid;
4-(N,N-dimethylamino)-1-hydroxybutylidene-1,1-bisphosphonic acid;
3-amino-1-hydroxypropylidene-1,1-bisphosphonic acid;
3-(N,N-dimethylamino)-1-hydroxypropylidene-1,1-bisphosphonic acid;
1-hydroxy-3-(N-methyl-N-pentylamino)propylidene- 1,1-bisphosphonic acid;
1-hydroxy-2-[3-pyridyl]ethylidene-1,1-bisphosphonic acid; and
4-(hydroxymethylene-1,1-bisphosphonic acid)piperidine;
or a pharmaceutically acceptable salt thereof;
wherein the dosage form is obtainable by the process of any one of Claims 1 to 16.

18. A pharmaceutical composition which is a tablet obtainable by wet granulation comprising by weight, 0.5 to 25% by weight of an active ingredient, which is the binder, selected from the group consisting of:
4-amino-1-hydroxybutylidene-1,1-bisphosphonic acid;
N-methyl-4-amino-1-hydroxybutylidene-1,1-bisphosphonic acid;
4-(N,N-dimethylamino)-1-hydroxybutylidene-1,1-bisphosphonic acid;
3-amino-1-hydroxypropylidene-1,1-bisphosphonic acid;
3-(N,N-dimethylamino)-1-hydroxypropylidene-1,1-bisphosphonic acid;
1-hydroxy-3-(N-methyl-N-pentylamino)propylidene-1,1-bisphosphonic acid;
1-hydroxy-2-[3-pyridyl]ethylidene-1,1-bisphosphonic acid; and
4-(hydroxymethylene-1,1-bisphosphonic acid)piperidine;
or a pharmaceutically acceptable salt thereof;
and from 30 to 70 % by weight of anhydrous lactose or hydrous fast flow lactose; 30 to 50 % by weight of microcrystalline cellulose, and 0.1 to 2% by weight magnesium stearate; with the proviso that the composition does not further comprise croscarmellose sodium.

19. The pharmaceutical composition of Claim 17 or 18 wherein the active ingredient is 4-amino-1-hydroxybutylidene-1,1-bisphosphonic acid.

20. The pharmaceutical composition of Claim 17 or 18 wherein the active ingredient is 4-amino-1-hydroxybutylidene-1,1-bisphosphonic acid monosodium salt.

21. The pharmaceutical composition of Claim 17 or 18 wherein the active ingredient is 4-amino-1-hydroxybutylidene-1,1-bisphosphonic acid monosodium salt trihydrate.

## Patentansprüche

1. Ein Verfahren zur Herstellung einer Tablette, die einen Wirkstoff enthält, der das Bindemittel ist, ausgewählt aus der Gruppe, bestehend aus:
4-Amino-1-hydroxybutyliden-1,1-bisphosphonsäure,
N-Methyl-4-amino-1-hydroxybutyliden-1,1-bisphosphonsäure,
4-(N,N-Dimethylamino)-1-hydroxybutyliden-1,1-bisphosphonsäure,
3-Amino-1-hydroxypropyliden-1,1-bisphosphonsäure,
3-(N,N-Dimethylamino)-1-hydroxypropyliden-1,1-bisphosphonsäure,
1-Hydroxy-3-(N-methyl-N-pentylamino)propyliden-1,1-bisphosphonsäure,
1-Hydroxy-2-[3-pyridyl]ethyliden-1,1-bisphosphonsäure und
4-(Hydroxymethylen-1,1-bisphosphonsäure)piperidin,
oder einem pharmazeutisch annehmbaren Salz davon,
wobei das Verfahren umfaßt:
(1) Bilden einer Pulvermischung aus dem Wirkstoff mit Verdünnungsmitteln,
(2) Naßgranulieren der Pulvermischung mit Wasser, um Körnchen zu bilden,
(3) Trocknen der Körnchen, um Wasser zu entfernen, und
(4) Verpressen der getrockneten Körnchenmischung zu einer erwünschten Tablettenform,
mit der Maßgabe, daß, wenn der Wirkstoff ein 5-mg-Äquivalent von 4-Amino-1-hydroxybutyliden-1,1-bisphosphonsäure ist, die Verdünnungsmittel wasserfreie Lactose und mikrokristalline Cellulose sind und der Trocknungsschritt in einem Gebläselufttrockner durchgeführt wird, die resultierenden Körnchen dann nicht mit Croscarmellose-Natrium und Magnesiumstearat vermischt werden.

2. Das Verfahren nach Anspruch 1, bei dem der Wirkstoff 4-Amino-1-hydroxybutyliden-1,1-bisphosphonsäure ist.

3. Das Verfahren nach Anspruch 1, bei dem der Wirkstoff 4-Amino-1-hydroxybutyliden-1,1-bisphosphonsäure-Mononatriumsalz ist.

4. Das Verfahren nach Anspruch 1, bei dem der Wirkstoff 4-Amino-1-hydroxybutyliden-1,1-bisphosphonsäure-Mononatriumsalz-Trihydrat ist.

5. Ein Verfahren gemäß Anspruch 1, 2, 3 oder 4, wobei das Verfahren umfaßt:
(1) Bilden einer Pulvermischung aus dem Wirkstoff mit Verdünnungsmitteln, 3 bis 25 Minuten lang, wobei ein Mischer, wie z.B. ein Planetengranulator oder ein Granulator mit hoher Scherkraft verwendet wird,
(2) Naßgranulieren der Pulvermischung durch Zugabe von Wasser unter Mischen über einen Zeitraum von 2 bis 30 Minuten, um Körnchen zu bilden,
(3) Trocknen der Körnchen, um Wasser zu entfernen, durch Verwendung erwärmter Luft, 10 Minuten bis 24 Stunden lang,
(4) Mahlen der getrockneten Körnchen zu einer einheitlichen Größe,
(5) Zugeben eines Sprengmittels zu und Vermischen eines Sprengmittels mit den getrockneten gemahlenen Teilchen, 2 bis 30 Minuten lang,
(6) Zugeben eines Gleitmittels zu und Vermischen eines Gleitmittels mit der Mischung, die das Sprengmittel enthält, 30 Sekunden bis 20 Minuten lang, und
(7) Verpressen der getrockneten Körnchenmischung zu einer gewünschten Tablettenform.

6. Das Verfahren nach Anspruch 5, bei dem die Verdünnungsmittel ausgewählt sind aus: Lactose, mikrokristalliner Cellulose, Calciumphosphat, Mannit, pulverförmiger Cellulose und vorgelierter Stärke.

7. Das Verfahren nach Anspruch 6, bei dem die Verdünnungsmittel Lactose und mikrokristalline Cellulose sind.

8. Das Verfahren nach Anspruch 7, bei dem die Lactose wasserfreie Lactose NF ist und die mikrokristalline Cellulose Avicel PH101 ist.

9. Das Verfahren nach Anspruch 5, bei dem das Sprengmittel ausgewählt ist aus der Gruppe, bestehend aus modifizierter Stärke, modifiziertem Cellulosepolymer und Croscarmellose-Natrium oder einer Kombination davon.

10. Das Verfahren nach Anspruch 9, bei dem das Sprengmittel Croscarmellose-Natrium ist.

11. Das Verfahren nach Anspruch 10, bei dem das Sprengmittel Croscarmellose-Natrium NF Typ A ist.

12. Das Verfahren nach Anspruch 5, bei dem das Gleitmittel ausgewählt ist aus der Gruppe, bestehend aus Magnesiumstearat, Calciumstearat, Stearinsäure, Natriumlaurylsulfat, Propylenglycol, Natriumdodecansulfonat, Natriumoleatsulfonat, mit Stearaten und Talk vermischtem Natriumlaurat und Natriumstearylfumarat.

13. Das Verfahren nach Anspruch 12, bei dem das Gleitmittel Magnesiumstearat ist.

14. Das Verfahren nach Anspruch 5, umfassend die Schritte:
(1) Bilden einer Pulvermischung aus 4-Amino-1-hydroxybutyliden-1,1-bisphosphonsäure, mikrokristalliner Cellulose und Lactose mit einem Granulator mit hoher Scherkraft, 3 bis 5 Minuten lang,
(2) Naßgranulieren der Pulvermischung durch die Zugabe von Wasser unter Mischen über einen Zeitraum von 3 bis 5 Minuten, um mit dem Scherkraftgranulator Körnchen zu bilden,
(3) Trocknen der Körnchen, um Wasser zu entfernen, durch Verwendung erwärmter Luft durch 10 Minuten bis 1 Stunde langes Trocknen mit einem Fließbetttrockner oder 12 bis 24 Stunden langes Trocknen in einem Plattentrockner,
(4) Mahlen der getrockneten Körnchen zu einer einheitlichen Größe unter Verwendung einer Hammermühle,
(5) Zugeben des Sprengmittels Croscarmellose-Natrium NF Typ A zu und Vermischen des Sprengmittels Croscarmellose-Natrium NF Typ A mit den getrockneten gemahlenen Teilchen, 3 bis 8 Minuten lang,
(6) Zugeben von Magnesiumstearat-Gleitmittel zu und Vermischen von Magnesiumstearat-Gleitmittel mit der Mischung, die das Sprengmittel Croscarmellose-Natrium NF Typ A enthält, mit einem Bandmischer oder einem Planetenmischer, 3 bis 8 Minuten lang,
(7) Verpressen der mit Gleitmittel versetzten Körnchenmischung zu einer erwünschten Tablettenform und
(8) Entstauben und Lagern der Tabletten.

15. Das Verfahren nach Anspruch 5, umfassend die Schritte:
(1) Bilden einer Pulvermischung aus 4-Amino-1-hydroxybutyliden-1,1-bisphosphonsäure, mikrokristalliner Cellulose und Lactose mit einem Planetengranulator, 10 bis 25 Minuten lang,
(2) Naßgranulieren der Pulvermischung durch die Zugabe von Wasser unter Mischen über einen Zeitraum von 3 bis 10 Minuten, um mit dem Planetengranulator Körnchen zu bilden,
(3) Trocknen der Körnchen, um Wasser zu entfernen, durch Verwendung erwärmter Luft durch 10 Minuten bis 1 Stunde langes Trocknen mit einem Fließbetttrockner oder 12-24 Stunden langes Trocknen in einem Plattentrockner,
(4) Mahlen der getrockneten Körnchen zu einer einheitlichen Größe unter Verwendung einer Hammermühle,
(5) Zugeben des Sprengmittels Croscarmellose-Natrium NF Typ A zu und Vermischen des Sprengmittels Croscarmellose-Natrium NF Typ A mit den getrockneten gemahlenen Teilchen, 3 bis 8 Minuten lang,
(6) Zugeben von Magnesiumstearat-Gleitmittel zu und Vermischen von Magnesiumstearat-Gleitmittel mit der Mischung, die das Sprengmittel Croscarmellose-Natrium NF Typ A enthält, mit einem Bandmischer oder einem Planetengranulator, 3 bis 8 Minuten lang,
(7) Verpressen der mit Gleitmittel versetzten Körnchenmischung zu einer erwünschten Tablettenform und
(8) Entstauben und Lagern der Tabletten.

16. Das Verfahren nach Anspruch 5, umfassend die Schritte:
(1) Bilden einer Pulvermischung aus 4-Amino-1-hydroxybutyliden-1,1-bisphosphonsäure, mikrokristalliner Cellulose Avicel PH101 und Lactose mit einem Granulator mit hoher Scherkraft, 3 bis 5 Minuten lang,
(2) Naßgranulieren der Pulvermischung durch die Zugabe von Wasser unter Mischen über einen Zeitraum von 3 bis 5 Minuten mit einem Granulator mit hoher Scherkraft, um Körnchen zu bilden,
(3) Trocknen der Körnchen, um Wasser zu entfernen, durch Verwendung erwärmter Luft, 10 Minuten bis eine Stunde lang, unter Verwendung eines Fließbetttrockners,
(4) Mahlen der getrockneten Körnchen zu einer einheitlichen Größe unter Verwendung einer Hammermühle,
(5) Zugeben des Sprengmittels croscarmellose-Natrium NF Typ A zu and Vermischen des Sprengmittels Croscarmellose-Natrium NF Typ A mit den getrockneten gemahlenen Teilchen, 3 bis 8 Minuten lang,
(6) Zugeben von Magnesiumstearat-Gleitmittel zu und Vermischen von Magnesiumstearat-Gleitmittel mit der Mischung, die das Sprengmittel Croscarmellose-Natrium NF Typ A enthält, mit einem Bandmischer, 3 bis 8 Minuten lang,
(7) Verpressen der mit Gleitmittel versetzten Körnchenmischung zu einer erwünschten Tablettenform und
(8) Entstauben und Lagern der Tabletten.

17. Eine feste Dosisform, die einen Wirkstoff enthält, ausgewählt aus der Gruppe, bestehend aus:
4-Amino-1-hydroxybutyliden-1,1-bisphosphonsäure,
N-Methyl-4-amino-1-hydroxybutyliden-1,1-bisphosphonsäure,
4-(N,N-Dimethylamino)-1-hydroxybutyliden-1,1-bisphosphonsäure,
3-Amino-1-hydroxypropyliden-1,1-bisphosphonsäure,
3-(N,N-Dimethylamino)-1-hydroxypropyliden-1,1-bisphosphonsäure,
1-Hydroxy-3-(N-methyl-N-pentylamino)propyliden-1,1-bisphosphonsäure,
1-Hydroxy-2-[3-pyridyl]ethyliden-1,1-bisphosphonsäure und
4-(Hydroxymethylen-1,1-bisphosphonsäure)piperidin,
oder einem pharmazeutisch annehmbaren Salz davon,
wobei die Dosisform durch das Verfahren nach irgendeinem der Ansprüche 1 bis 16 erhältlich ist.

18. Eine pharmazeutische Zusammensetzung, die eine durch Naßgranulieren erhältliche Tablette ist, umfassend, bezogen auf das Gewicht, 0,5 bis 25 Gew.-% eines Wirkstoffs, der das Bindemittel ist, ausgewählt aus der Gruppe, bestehend aus:
4-Amino-1-hydroxybutyliden-1,1-bisphosphonsäure,
N-Methyl-4-amino-1-hydroxybutyliden-1,1-bisphosphonsäure,
4-(N,N-Dimethylamino)-1-hydroxybutyliden-1,1-bisphosphonsäure,
3-Amino-1-hydroxypropyliden-1,1-bisphosphonsäure,
3-(N,N-Dimethylamino)-1-hydroxypropyliden-1,1-bisphosphonsäure,
1-Hydroxy-3-(N-methyl-N-pentylamino)propyliden-1,1-bisphosphonsäure,
1-Hydroxy-2-[3-pyridyl]ethyliden-1,1-bisphosphonsäure und
4-(Hydroxymethylen-1,1-bisphosphonsäure)piperidin,
oder einem pharmazeutisch annehmbaren Salz davon,
und 30 bis 70 Gew.-% wasserfreie Lactose oder wäßrige schnellfließende Lactose, 30 bis 50 Gew.-% mikrokristalline Cellulose und 0,1 bis 2 Gew.-% Magnesiumstearat, mit der Maßgabe, daß die Zusammensetzung darüber hinaus nicht Croscarmellose-Natrium enthält.

19. Die pharmazeutische Zusammensetzung nach Anspruch 17 oder 18, wobei der Wirkstoff 4-Amino-1-hydroxybutyliden-1,1-bisphosphonsäure ist.

20. Die pharmazeutische Zusammensetzung nach Anspruch 17 oder 18, wobei der Wirkstoff 4-Amino-1-hydroxybutyliden-1,1-bisphosphonsäure-Mononatriumsalz ist.

21. Die pharmazeutische Zusammensetzung nach Anspruch 17 oder 18, wobei der Wirkstoff 4-Amino-1-hydroxybutyliden-1,1-bisphosphonsäure-Mononatriumsalz-Trihydrat ist.

## Revendications

1. Procédé pour la préparation d'un comprimé contenant un ingrédient actif qui est le liant, choisi dans le groupe constitué par :
• l'acide 4-amino-1-hydroxybutylidéne-1,1-bis-phosphonique ;
• l'acide N-méthyl-4-amino-1-hydroxybutylidène-1,1-bisphosphonique ;
• l'acide 4-(N,N-diméthylamino)-1-hydroxy-butylidène-1,1-bisphosphonique ;
• l'acide 3-amino-1-hydroxypropylidène-1,1-bis-phosphonique ;
• l'acide 3-(N,N-diméthylamino)-1-hydroxy-propylidène-1,1-bisphosphonique ;
• l'acide 1-hydroxy-3-(N-méthyl-N-pentylamino) propylidène-1,1-bisphosphonique ;
• l'acide 1-hydroxy-2-[3-pyridyl]-éthylidène-1,1-bisphosphonique ; et
• la pipéridine de 4-(acide hydroxyméthyléne-1,1-bisphosphonique) ;
ou un sel pharmaceutiquement acceptable de ceux-ci, lequel procédé comprend les étapes consistant à :
(1) former un mélange en poudre de l'ingrédient actif avec des diluants,
(2) effectuer une granulation par voie humide du mélange en poudre avec de l'eau pour former des granulés,
(3) sécher les granulés pour enlever l'eau, et
(4) compresser le mélange de granulés lubrifiés en une forme de comprimé désirée ; pourvu que quand l'ingrédient actif est équivalent à 5 mg d'acide 4-amino-1-hydroxybutylidéne-1,1-bis-phosphonique, les diluants sont le lactose anhydre et la cellulose microcristalline, et l'étape de séchage est mise en oeuvre dans un séchoir à air forcé, puis les granulés obtenus ne sont pas mélangés avec la croscarmellose sodique et le stéarate de magnésium.

2. Procédé selon la revendication 1, dans lequel l'ingrédient actif est l'acide 4-amino-1-hydroxy-butylidène-1,1-bisphosphonique.

3. Procédé selon la revendication 1, dans lequel l'ingrédient actif est le sel monosodique d'acide 4-amine-1-hydroxybutylidéne-1,1-bis-phosphonique.

4. Procédé selon la revendication 1, dans lequel l'ingrédient actif est le sel monosodique d'acide 4-amino-1-hydroxybutylidéne-1,1-bis-phosphonique trihydraté.

5. Procédé selon la revendication 1, 2, 3 ou 4, lequel procédé comprend :
(1) former un mélange en poudre d'un ingrédient actif avec les diluants pendant 3 à 25 minutes, en utilisant un agitateur tel qu'un de type planétaire ou un granulateur à cisaillement élevé,
(2) effectuer une granulation par voie humide du mélange en poudre, en ajoutant de l'eau tout en mélangeant pendant une période de 2 à 30 minutes pour former des granulés,
(3) sécher les granulés pour enlever l'eau au moyen d'air chauffé pendant 10 minutes à 24 heures,
(4) moudre les granulés secs en une taille uniforme,
(5) ajouter et mélanger un agent de désintégration avec les particules séchées moulues pendant 2 à 30 minutes,
(6) ajouter et mélanger un lubrifiant au mélange contenant l'agent de désintégration pendant 30 secondes à 20 minutes, et
(7) compresser le mélange de granulés séché en une forme de comprimé désirée.

6. Procédé selon la revendication 5, dans lequel les diluants sont choisis parmi : le lactose, la cellulose microcristalline, le phosphate de calcium, le mannitol, la cellulose en poudre, l'amidon préalablement mis en gélatine.

7. Procédé selon la revendication 6, dans lequel les diluants sont le lactose et la cellulose microcristalline.

8. Procédé selon la revendication 7, dans lequel le lactose est le lactose anhydre NF, et la cellulose microcristalline est l'Avicel PH101.

9. Procédé selon la revendication 5, dans lequel l'agent de désintégration est choisi dans le groupe constitué par l'amidon modifié, le polymère de cellulose modifié et la croscarmellose sodique ou une combinaison de ceux-ci.

10. Procédé selon la revendication 9, dans lequel l'agent de désintégration est la croscarmellose sodique.

11. Procédé selon la revendication 10, dans lequel l'agent de désintégration est la croscarmellose sodique NF de type A.

12. Procédé selon la revendication 5, dans lequel le lubrifiant est choisi dans le groupe constitué par le stéarate de magnésium, le stéarate de calcium, l'acide stéarique, le lauryle sulfate de sodium, le propylène glycol, le dodécane sulfonate de sodium, l'oléate sulfonate de sodium et le laurate de sodium mélangé avec des stéarates et le talc, et le stéaryle fumarate.

13. Procédé salon la revendication 12, dans lequel le lubrifiant est le stéarate de magnésium.

14. Procédé selon la revendication 5, qui comprend les étapes consistant à :
(1) former un mélange en poudre d'acide 4-amino-1-hydroxybutylidène-1,1-bisphosphonique, de cellulose microcristalline et de lactose avec un granulateur à cisaillement élevé pendant 3 à 5 minutes,
(2) effectuer une granulation par voie humide du mélange en poudre, en ajoutant de l'eau tout en mélangeant pendant une période de 3 à 5 minutes pour former des granulés avec le granulateur à cisaillement élevé,
(3) sécher les granulés pour enlever l'eau au moyen d'air chauffé en séchant pendant 10 minutes à 1 heure dans un lit fluide, ou 12 à 24 heures dans un dessiccateur à plateaux,
(4) moudre les granulés secs en une taille uniforme en utilisant un broyeur de type marteaux,
(5) ajouter et mélanger l'agent de désintégration croscarmellose sodique NF de type A avec les particules séchées moulues pendant 3 à 8 minutes,
(6) ajouter et mélanger le lubrifiant stéarate de magnésium au mélange contenant l'agent de désintégration croscarmellose sodique NF de type A avec un mélangeur à ruban ou un agitateur planétaire pendant 3 à 8 minutes,
(7) compresser le mélange de granulés lubrifié en une forme de comprimé désirée, et
(8) dépoussiérer et stocker les comprimés.

15. Procédé selon la revendication 5, qui comprend les étapes consistant à :
(1) former un mélange en poudre d'acide 4-amino-1-hydroxybutylidène-1,1-bisphosphonique, de cellulose microcristalline et de lactose avec un granulateur planétaire pendant 10 à 25 minutes,
(2) effectuer une granulation par voie humide du mélange en poudre, en ajoutant de l'eau, tout en mélangeant pendant une période de 3 à 10 minutes pour former des granulés avec le granulateur planétaire,
(3) sécher les granulés pour enlever l'eau au moyen d'air chauffé en séchant pendant 10 minutes à 1 heure dans un lit fluide, ou 12 à 24 heures dans un dessiccateur à plateaux,
(4) moudre les granulés secs en une taille uniforme en utilisant un broyeur de type marteaux,
(5) ajouter et mélanger l'agent de désintégration croscarmellose sodique NF de type A avec les particules séchées moulues pendant 3 à 8 minutes,
(6) ajouter et mélanger le lubrifiant stéarate de magnésium au mélange contenant l'agent de désintégration croscarmellose sodique NF de type A avec un mélangeur à ruban ou un granulateur planétaire pendant 3 à 8 minutes, et
(7) compresser le mélange de granulés lubrifié en une forme de comprimé désirée, et
(8) dépoussiérer et stocker les comprimés.

16. Procédé selon la revendication 5, qui comprend les étapes consistant à :
(1) former un mélange en poudre d'acide 4-amino-1-hydroxybutylidène-1,1-bisphosphonique, de cellulose microcristalline Avicel PH101 et de lactose avec un granulateur à cisaillement élevé pendant 3 à 5 minutes,
(2) effectuer une granulation par voie humide du mélange en poudre, en ajoutant de l'eau, tout en mélangeant pendant une période de 3 à 5 minutes pour former des granulés avec un granulateur à cisaillement élevé,
(3) sécher les granulés pour enlever l'eau au moyen d'air chauffé en séchant pendant 10 minutes à une heure en utilisant un séchoir à lit fluide,
(4) moudre les granulés secs en une taille uniforme en utilisant un broyeur de type marteaux,
(5) ajouter et mélanger l'agent de désintégration croscarmellose sodique NF de type A avec les particules séchées moulues pendant 3 à 8 minutes,
(6) ajouter et mélanger le lubrifiant stéarate de magnésium au mélange contenant l'agent de désintégration croscarmellose sodique NF de type A avec un mélangeur à ruban pendant 3 à 8 minutes,
(7) compresser le mélange de granulés lubrifié en une forme de comprimé désirée, et
(8) dépoussiérer et stocker les comprimés.

17. Forme de posologie solide contenant un ingrédient actif choisi dans le groupe constitué par :
• l'acide 4-amino-1-hydroxybutylidène-1,1-bis-phosphonique ;
• l'acide N-méthyl-4-amino-1-hydroxybutylidène-1,1-bisphosphonique ;
• l'acide 4-(N,N-diméthylamino)-1-hydroxy-butylidéne-1,1-bisphosphonique ;
• l'acide 3-amino-1-hydroxypropylidène-1,1-bis-phosphonique ;
• l'acide 3-(N,N-diméthylamino)-1-hydroxypropylidène-1,1-bisphosphonique ;
• l'acide 1-hydroxy-3-(N-méthyl-N-pentylamino) propylidène-1,1-bisphosphonique ;
• l'acide 1-hydroxy-2-[3-pyridyl] éthylidène-1,1-bisphosphonique ; et
• la pipéridine de 4-(acide hydroxyméthylène-1,1-bisphosphonique) ;
• ou un sel pharmaceutiquement acceptable de ceux-ci ;
dans lequel la forme de posologie peut être obtenue par les procédés selon l'une quelconque des revendications 1 à 16.

18. Composition pharmaceutique qui est un comprimé que l'on peut obtenir par granulation par voie humide comprenant en poids, 0,5 à 25 % en poids d'un ingrédient actif qui est le liant, choisi dans le groupe constitué par :
• l'acide 4-amino-1-hydroxybutylidène-1,1-bis-phosphonique ;
• l'acide N-méthyl-4-amino-1-hydroxybutylidène-1,1-bisphosphonique ;
• l'acide 4-(N,N-diméthylamino)-1-hydroxybutylidène-1,1-bisphosphonique ;
• l'acide 3-amino-1-hydroxypropylidène-1,1-bisphosphonique ;
• l'acide 3-(N,N-diméthylamino)-1-hydroxypropylidéne-1,1-bisphosphonique ;
• l'acide 1-hydroxy-3-(N-méthyl-N-pentylamino) propylidène-1,1-bisphosphonique ;
• l'acide 1-hydroxy-2-[3-pyridyl]-éthylidène-1, 1-bisphosphonique ; et
• la pipéridine de 4-(acide hydroxyméthylène-1, 1-bisphosphonique)
• ou un sel pharmaceutiquement acceptable de ceux-ci ;
et de 30 à 60 % en poids de lactose anhydre ou de lactose hydrique à flux rapide ; 30 à 50 % en poids de cellulose microcristalline et 0,1 à 2 % en poids de stéarate de magnésium, pourvu que la composition ne comprenne pas en outre de croscarmellose sodique.

19. Composition pharmaceutique selon la revendication 17 ou 18, dans lequel l'ingrédient actif est l'acide 4-amino-1-hydroxybutylidène-1,1-bis-phosphonique.

20. Composition pharmaceutique selon la revendication 17 ou 18, dans lequel l'ingrédient actif est le sel monosodique d'acide 4-amino-1-hydroxybutylidène-1,1-bisphosphonique.

21. Composition pharmaceutique selon la revendication 17 ou 18, dans lequel l'ingrédient actif est le sel monosodique d'acide 4-amino-1-hydroxybutylidène-1,1-bisphosphonique trihydraté.
